(19)

Europäisches
Patentamt
European
Patent Office
Office européen
des brevets

(11) **EP 2 359 900 A2**

(12) **EUROPEAN PATENT APPLICATION**
published in accordance with Art. 153(4) EPC

(43) Date of publication:
**24.08.2011 Bulletin 2011/34**

(21) Application number: **09823889.2**

(22) Date of filing: **28.10.2009**

(51) Int Cl.:
*A61N 5/00* (2006.01)  *A61N 7/00* (2006.01)

(86) International application number:
**PCT/RU2009/000581**

(87) International publication number:
**WO 2010/050849 (06.05.2010 Gazette 2010/18)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **29.10.2008 RU 2008142701**

(71) Applicant: **Rasnetsov, Lev Davidovich Nizhny Novgorod 603000 (RU)**

(72) Inventors:
• **SHVARTSMAN, Iakov Yudelevich Nizhny Novgorod 603105 (RU)**

• **CHEREPENNIKOV, Vladimir Vasilyevich Nizhny Novgorod 603043 (RU)**
• **MUDROV, Vladimir Mikhailovich Nizhny Novgorod 603108 (RU)**
• **VINOKUROV, Mikhail Evgenyevich Nizhny Novgorod 603086 (RU)**
• **RASNETSOV, Lev Davidovich Nizhny Novgorod 603000 (RU)**

(74) Representative: **HOFFMANN EITLE Patent- und Rechtsanwälte Arabellastraße 4 81925 München (DE)**

(54) **METHOD FOR CAPTURING THE CHANGES INDUCED IN AN INITIAL CRYSTAL STRUCTURE BY THE ACTION OF THE ACOUSTIC AND ELECTROMAGNETIC FIELDS OF A BIOLOGICAL MEDIUM AND A DEVICE FOR CARRYING OUT SAID METHOD, METHOD FOR DUPLICATING THE CHANGES IN THE INITIAL CRYSTAL STRUCTURE IN THE STRUCTURE OF A DUPLICATE CRYSTAL (VARIANTS) AND A DEVICE FOR CARRYING OUT SAID METHOD (VARIANTS), METHOD FOR EXERTING AN EFFECT ON A BIOLOGICAL OBJECT (VARIANTS) AND A DEVICE FOR CARRYING OUT SAID METHOD**

(57) The invention relates to medicinal equipment and is useful in physiotherapy for controlling the functions of biological objects.

A method for capturing the changes induced by the action of acoustic and electromagnetic fields of a biological medium using a crystal whose structure comprises at least one semiconductor junction is offered. The crystal is positioned on an insulating plate which is in direct contact with the biological medium, and the changes in the crystal structure are captured by means of exposing the semiconductor junction thereof to a cut-off polarity electric pulse. A method for duplicating the changes that occurred in the initial crystal structure in a duplicate crystal structure is **characterized in that** both crystals are positioned on one face of a water-filled hollow insulating cartridge; then the semiconductor junction of the initial crystal is repeatedly exposed to the action of a straight-polarity reproduction pulse, after which the semiconductor junction of the duplicate crystal is exposed to the action of a capture cut-off polarity pulse. Various methods of affecting a biological object by crystals with changed structure; a device for capturing the changes induced by the action of acoustic and electromagnetic fields of a biological medium in an initial crystal structure; a device for duplicating the changes that occurred in an initial crystal structure in a duplicate crystal structure; a device for causing an affect on a biological object comprising an exposure source in the form of a crystal in whose structure the changes induced by the action of acoustic and electromagnetic fields of a biological medium are captured. Ind. cl. 15; dep. cl. 21; Ill. 13.

EP 2 359 900 A2

## Description

## Field of the Art

The invention relates to medicinal equipment and is useful in physiotherapy for controlling the functions of biological objects.

## Background of the Invention

In a system of two tissue cultures separated from each other, cells are known to interact.

An experiment was carried out at the Moscow Institute for Cybernetic Medicine (see http:www/mricm.com/mricm/feedback/). The underlying idea of this experiment is the hypothesis advanced by A.Sh. Avshalumov that, upon destruction (or self-organization of the structure) of a biological object, the energy stored in high-organized links inside this object is released in the form of some emission that carries information about the properties of the object; under certain conditions, this emission can impact other biological objects inducing a tailored change in the properties of these objects, that is, playing the role of a controlling action. The setup represents a two-chamber air-tight system, one insulated chamber containing a biological substance, which is the exposure source object, and the other containing the receiver object, a biological substance the properties whereof are to be changed in the direction desired by the user by means of remote influence that appears in the moment when the structure of the source object changes (is destroyed). During the experiment monitored were temperature inside each chamber and pressure inside the source object chamber. The design of the device excludes energy transfer from the source object to the receiver object. Studies were carried out in collaboration with a number of leading research institutions of the Russian Academy of Sciences specializing in microbiology. These studies included experiments on $\beta$-cells (the stimulating effect of remote influence on the $\beta$-cell culture was demonstrate), Leydig cells (proliferative activity of the cells and testosterone producing function increased), and human stem cells (the main indicators of the cell activity increased). As judged from the results of these experiments, the authors discovered a remote control effect of one biological object on another, this effect being manifested as an induction of a tailored change in the properties of the latter.

Research in this field resulted in working out of a whole number of methods for capturing, on a carrier, of the intrinsic field of a biological medium in the period of mass death of its cells or in the period of an impact of stimulating factors that provide an intensive development and proliferation of cells, and in creating, on the basis of the aforementioned carrier, of active devices that allow the activity of identical cells to be controlled, specifically in a human body.

There is a method for causing wave action on pathogenic microorganisms, viruses, or tumor cells in an experiment (see the Russian Federation patent No. 2250788, A61N 5/00, 2005). The method is carried out with the help of a Gann-effect diode crystal as follows: the feed voltage is applied to the working volume of this crystal after preliminarily bringing it in contact with such a microorganism, or a virus, or a tumor cell, and then the crystal is switched off and is positioned at the acupressure points and zones of a mammal. For preliminarily bringing the crystal in contact, used are the intrinsic oscillations of a pathogenic microorganism, or a virus, or a tumor cell during the period of their mass death under the impact of an inhibiting factor, such as antibiotics, or sterilizing agents, or thermal impact, or ultraviolet light, or radiation. Further, for preliminary contact useful are the intrinsic oscillations of an autochthonic microorganism of a mammal in the period it is under the impact of a favorable stimulating factor that provides the intensive development and proliferation of the autochthonic microorganism in the logarithmic growth phase, such as bifidogenic or lactogenic growth stimulators, vitamins, and microelements. The wave emitted from the Gann-effect diode comes to the object (microorganism), is modulated by the intrinsic oscillations thereof, is reflected back, and comes to the diode. With the power supply being switched off, the crystal structure of the diode is formed under the action of the reflected electromagnetic wave. A drawback of the method consists of the low efficiency of the resulting carrier on account of the fact that the nonlinear interactions of the Gann-effect diode with the intrinsic oscillations of the object are required for modulation to occur, but these interactions, if any exist, are negligible, with the modulation depth being accordingly low. The effect of the wave reflected from the object onto the Gann-effect diode structure is negligible compared to the background radiation.

There is a device for changing the activity of a biological cell (see the Russian Federation Patent No. 2055604, 1993) comprising an active unit to carry out the initial receipt and subsequent transmission of electromagnetic waves, this unit being positioned in the immediate vicinity of a biological cell. The active unit is designed as a single unit and memorizes received electromagnetic waves. To the active unit, attached is a temperature-changing unit. As the single unit, used is a crystal of a semiconductor device; as the temperature-changing unit, used is at least one semiconductor junction of this device with a power supply source connected thereto. As the semiconductor device, the invention uses a transistor, the emitter whereof is directly connected to the power supply source and the collector through a switch, wherein the transistor base is connected to the collector thereof via a resistor. The device is equipped with a shielding housing and a cover for protecting the data stored in the device memory from external impacts.

The device operates in the following manner. First, information about the metabolic activity of deposited cells is recorded to the memory unit, being transformed by any of the known methods. The temperature-changing unit performs heating of the single unit. With this, the information about the changed metabolic activity of the deposited cells is recorded to the memory unit at the bioresonance frequency. Heating is followed by forced or natural cooling. Cooling may precede heating, which constitutes the temperature variation cycle of the single unit. Then, a patient carrying pathogenic microorganisms whose activity is to be changed is positioned in the immediate vicinity of the single unit, and the temperature variation cycle is carried out. The oscillator emits low-intensity electromagnetic waves at the bioresonance frequency of the cells. As a result of exposure, the metabolic activity of the exposed cell is established at the level analogous to the deposited cell which has a changed metabolic activity. In order for the tailored metabolic activity in the exposed cells to be maintained for a long period of time, repeated exposure sessions are carried out in certain time intervals.

Multiple experimental checks of the recording method according to the Russian Federation patent No. 2055604 showed that the device does not provide the inactivation of pathogenic microorganisms and does not provide efficient elimination of various pathogenic and conditionally pathogenic microorganisms occurring in a human or animal body. The low efficiency of the device arises primarily from the weak interactions of an inactivated strain with the surrounding medium in the course of recording. A reduced metabolic activity of a cell means reduced rates of metabolism, which is the major energy source in a cell, naturally leading to a reduction in all external energy manifestations of the cell, that is, electromagnetic fields to be recorded, thereby controlling the recording level. Another drawback consists of the shielding housing and cover, which not only make the record inefficient but also inhibit the subsequent therapeutic effect. Further, data recording and data readout during the therapeutic action both occur in the identical manner. If the crystal carries any information about the inactivated strain, then apart from the reproduction of the recorded data, the absent (under shielding) external field should be recorded during each healing session; that is, the crystal should enter its natural state rather than storing the preceding record.

With respect to the problem to be approached, the most pertinent piece of prior art for the present invention is a method for recording a polarized electromagnetic emission from an inactivated pathogenic strain of microorganisms on a crystal and a device for carrying out this method, a method for changing the activity of a strain of pathogenic microorganisms and a device for carrying out this method, and a method for eliminating a strain of pathogenic microorganisms from a man or an animal according to the Russian Federation patent No. 2199356,2000.

The recording method comprises inactivating a deposited pathogenic strain of microorganisms in a test tube, positioning the tube in the immediate vicinity to a means for recording, reproducing the emission spectra of biological cells, and carrying out a temperature variation cycle for the recording and reproducing means in the range from 10 to 400°C. While temperature changes, a polarized electromagnetic emission having a wave length in the range from 3 to 100 $\mu$m from an inactivated pathogenic microorganism strain is recorded on the means-With this, steady energy states are formed to endow the means for recording and reproducing the emission spectra of biological cells with the ability to emit analogous polarized electromagnetic waves upon subsequent temperature variation cycles. The aforementioned means is selected from silicon, germanium, diamond, and gallium arsenide.

The aforementioned polarized electromagnetic emission from the means for recording and reproducing spectra is used for exposing pathogenic microorganisms and inactivating them in a human body. With the use of the means for changing the activation of pathogenic microorganisms positioned in the immediate vicinity of a patient, the temperature variation cycle is carried out for the means for recording and reproducing the emission spectra of biological cells whereon the polarized electromagnetic emission from the inactivated pathogenic microorganism is captured. The pathogenic strain is eliminated from a human or animal body via exposing the patient to the recorded electromagnetic emission for a period of I ms to 1000 s in a schedule of 1 to 48 sessions a day during a period of 3 days to 2 months.

The device for recording the polarized electromagnetic emission from an inactivated pathogenic microorganism strain comprises a working cell positioned in a vessel containing the inactivated pathogenic microorganism strain, this cell having at least one means for recording and reproducing the emission spectra of biological cells and a means for varying temperature. Further, the device comprises a control unit which is connected to the temperature variation means and an ancillary cell for restoring the level of polarized electromagnetic emission in the working cell, this ancillary cell being embodied in a similar way as the working cell and positioned in the vessel containing the strain. The device further comprises at least one reference cell intended for restoring the level of the polarized electromagnetic emission from the strain in the working and ancillary cells and a electromagnetic wave oscillator which is installed in the immediate vicinity of the vessel in which the strain is contained.

The device for changing the activity of a pathogenic microorganism strain comprises at least one working cell which has at least one means for recording and reproducing the emission spectra of biological cells on which the polarized electromagnetic emission of the strain is recorded, a control unit, at least one ancillary cell, one reference cell for restoring the level of the polarized electromagnetic emission, and a unit for erasing the polarized electromagnetic emission.

The recording method according to the Russian Federation patent No. 2199356 has a low recording level and, thereby, low efficiency on account of the crystal during recording being positioned immediately in the inactivated pathogenic microorganism strain, after which the temperature variation cycle is carried out for the means for recording and reproducing the emission spectrum of biological cells for a period of 1 ms to 1000 s in the range from 10 to 400°C (in examples, from 20 to 120°C). Since a semiconductor device crystal is used as the recording and reproducing means and the semiconductor junction of the same crystal is used as the temperature variation means, not only does this heating up of the crystal positioned in the culture destroy the semiconductor junction, but it also perturbs the electromagnetic fields of the inactivated strain. Further, the inactivated strain weakly interacts with the surrounding medium, and its fields are weak, too, which is responsible for the low recording level.

Disclosure of the Invention

One object of the invention is to create a method for capturing the changes induced by the action of acoustic and electromagnetic fields of a biological medium in an initial crystal structure providing a high level and quality of capturing and to create a device for carrying out this method.

Another object of the invention is to create a method for duplicating the changes that occurred in the initial crystal structure in a duplicate crystal structure with a high duplicating quality and to create a device for carrying out this method.

One more object of the invention is to create a method for causing an effect on a biological object, specifically a man or an animal, and a device for carrying out this method.

To attain these objects, we propose a group of inventions linked by a single inventive concept: a method for capturing the changes induced in the structure of an initial crystal by the action of acoustic and electromagnetic fields of a biological medium and a device for carrying out this method, a method for duplicating the changes that occurred in the initial crystal structure in the structure of a duplicate crystal (variants) and a device for carrying out this method (variants), and a method for causing an effect on a biological object (variants) and a device for carrying out this method (variants).

The objects are attained by the method for capturing the changes induced in the structure of an initial crystal by the action of acoustic and electromagnetic fields of a biological medium, wherein according to the invention a crystal whose structure comprises a least one semiconductor junction is used, the crystal is positioned on an insulating plate which is in direct contact with the biological medium placed in a container, and the changes in the crystal structure are captured via exposing the semiconductor junction thereof to a cut-off polarity electric pulse.

The method uses an insulating plate made of a material having an acoustic wave absorption coefficient of no greater than 30 dB/cm and an electromagnetic wave absorption coefficient of no greater than 0.1 Neper. The semiconductor junction of the crystal is exposed to a square electric pulse having an amplitude of 3 to 4 V and a duration of 0.1 to 1 second.

Further, according to the present invention, a biological medium is preliminarily exposed to an inactivating agent, and the excitation of the crystal structure is carried out at the peak level of the field of the biological medium which is determined experimentally for the type of biological medium used under the impact of the inactivating agent used.

The objects are further attained by a method for duplicating the changes that occurred in the initial crystal structure in a duplicate crystal structure, wherein according to the invention, crystals are used whose structure comprises at least one semiconductor junction wherein the changes induced by the effect of the acoustic and electromagnetic fields of the biological medium are captured in the initial crystal structure, wherein both crystals are positioned on one face of a water-filled hollow insulating cartridge; then the semiconductor junction of the initial crystal is repeatedly exposed to a straight-polarity reproduction pulse, after which the semiconductor junction of the duplicate crystal is exposed to a capture cut-off polarity pulse. The method employs an insulating cartridge made of a material having an acoustic wave absorption coefficient of no greater than 30 dB/cm and an electromagnetic wave absorption coefficient of no greater than 0.1 Neper. The semiconductor junction of the crystal is exposed to a square electric pulse having an amplitude of 3 to 4 V and a duration of 0.1 to 1 second.

The objects are further attained by a second variant of the method for duplicating the changes that occurred in an initial crystal structure in the duplicate crystal structure, wherein according to the invention crystals are used such that the structure thereof comprises at least one semiconductor junction, wherein the changes induced by the action of acoustic and electromagnetic fields of a biological medium are captured by the initial crystal structure, wherein the duplicate crystal is positioned on an insulating plate which is in direct contact with a liquid medium, the semiconductor junction of the initial crystal is repeatedly exposed to a straight-polarity reproduction pulse, the generated electric current oscillations modulate the light flux of a powerful semiconductor laser to expose the liquid medium placed in a container having a radial-symmetrical shape, after which the semiconductor junction of the duplicate crystal is exposed to a capture cut-off polarity pulse. The method employs an insulating plate made of a material having an acoustic wave absorption coefficient of no greater than 30 dB/cm and an electromagnetic wave absorption coefficient of no greater than 0.1 Neper. The semiconductor junction of the crystal is exposed to a square electric pulse having an amplitude of 3 to 4 V and a duration of 0.1 to 1 second. The preferred liquid medium is physiological saline.

The objects are also attained by a method for causing an affect on a biological object wherein according to the invention, the source of exposure is a crystal the structure whereof comprises at least one semiconductor junction, with the changes induced by the action of acoustic and electromagnetic fields of the biological medium being captured by the crystal structure, wherein the crystal is positioned on one face of a water-filled hollow insulating cartridge and another face is in direct contact with the biological object, and the semiconductor junction of the crystal is repeatedly exposed to a straight-polarity electric pulse. Further according to the invention, the light flux of a light-emitting diode, which is used to expose the aqueous medium, is modulated by electric current oscillations obtained at the crystal outlet. The method employs an insulating cartridge made of a material having an acoustic wave absorption coefficient of no greater than 30 dB/cm and an electromagnetic wave absorption coefficient of no greater than 0.1 Neper.

The objects are further attained by a method for causing art affect on a biological object, wherein according to the invention, the source of exposure is a crystal the structure whereof comprises at least one semiconductor junction, with the changes induced by the action of acoustic and electromagnetic fields of the biological medium being captured by the crystal structure, wherein the semiconductor junction of the crystal is repeatedly exposed to a reproduction straight-polarity electric pulse and the generated electric current oscillations modulate the light flux of a light-emitting diode directed to the biological object.

The objects are further attained by a variant of the method for causing an affect on a biological object, wherein according to the invention the source of exposure is a crystal the structure whereof comprises at least one semiconductor junction, with the changes induced by the action of acoustic and electromagnetic fields of the biological medium being captured by the crystal structure, wherein the semiconductor junction of the crystal is repeatedly exposed to a reproduction straight-polarity pulse, and the generated electric current oscillations modulate the light flux of a light-emitting diode directed to the biological object.

The objects are further attained by a variant of the method for causing an affect on a human or animal body, wherein according to the invention the biological medium that is the initiator of a pathologic process is first recognized, a crystal is used with the changes induced by the acoustic and electromagnetic fields of this biological medium being captured in the crystal structure, wherein the crystal is positioned on one face of a hollow insulating cartridge filled with an aqueous medium, wherein the cartridge is positioned on the skin of a man or animal in the area of projection of the pathologic organ, and repeated exposure is carried out in a schedule of twa to six sessions a day, 10 seconds per session, for at least five days. Further according to the invention, the electric current oscillations obtained at the crystal outlet modulate the light flux of a light-emitting diode directed to the biological object. The method uses an insulating cartridge made of a material having an acoustic wave absorption coefficient of no greater than 30 dB/cm and an electromagnetic wave absorption coefficient of no greater than 0.1 Neper.

The objects are further attained by a variant of the method for causing an affect on a human or animal body, wherein according to the invention the biological medium that is the initiator of a pathologic process is first recognized, a crystal is used with the changes induced by the acoustic and electromagnetic fields of this biological medium being captured in the crystal structure, wherein the semiconductor junction of the crystal is repeatedly exposed to a reproduction straight-polarity pulse, and the generated electric current oscillations modulate the light flux of a light-emitting diode directed to the skin of a man or an animal in the area of projection of the pathologic organ or to the surface of a hollow organ, and exposure is carried out in a schedule of two to six sessions a day, 10 seconds per session, for at least five days.

The objects are further attained by a variant of the method for causing an affect on a human or animal body, wherein according to the invention the biological medium that is the initiator of a pathologic process is first recognized, a crystal is used with the changes induced by the acoustic and electromagnetic fields of this biological medium being captured in the crystal structure, wherein the semiconductor junction of the crystal is repeatedly exposed to a reproduction straight-polarity polarity pulse, the generated electric current oscillations modulate the light flux of a light-emitting diode directed to the skin of the man or an animal in the area of projection of the pathologic organ or to the surface of a hollow organ, and exposure is carried out in a schedule of two to six sessions a day, 10 seconds per session, for at least five days.

The objects are further attained by a device for capturing the changes in the initial crystal structure induced by the action of acoustic and electromagnetic fields of the biological medium comprising at least one semiconductor junction, this device according to the invention comprising a capture pulse generator the outlet whereof is connected in cut-off polarity to the semiconductor junction of the crystal, the crystal being positioned on an insulating plate which is in contact with the biological medium placed in a container. The insulating plate is made of a material having an acoustic wave absorption coefficient of no greater than 30 dB/cm and an electromagnetic wave absorption coefficient of no greater than 0.1 Neper, for example, of radio frequency ceramics or fluoroplastic.

The objects are further attained by a device for duplicating the changes that occurred in an initial crystal structure in a duplicate crystal structure, the device according to the invention comprising crystals the structure whereof comprises at least one semiconductor junction, wherein the changes induced by the action of acoustic and electromagnetic fields of the biological medium are captured in the initial crystal structure, a reproduction pulse generator connected in straight polarity to the semiconductor junction of the initial crystal, a capture pulse generator connected in cut-off polarity to the semiconductor junction of the duplicate crystal, wherein the crystals are positioned on one face of a hollow insulating cartridge filled with an aqueous medium. The insulating cartridge is made of a material having an acoustic wave absorption coefficient of no greater than 30 dB/cm and an electromagnetic wave absorption coefficient of no greater than 0.1 Neper, for example, radioengineering ceramics of fluoroplastic.

The objects are further attained by a variant of the device for duplicating the changes that occurred in an initial crystal structure in a duplicate crystal structure, the device according to the invention comprising crystals the structure whereof comprises at least one semiconductor junction, wherein the changes induced by the acoustic and electromagnetic fields of the biological medium are captured in the initial crystal structure; a reproduction pulse generator connected in straight polarity through a power amplifier to the semiconductor junction of the initial crystal the outlet whereof is connected to a powerful semiconductor laser; a capture pulse generator connected in cut-off polarity to the semiconductor junction of the duplicate crystal, wherein the duplicate crystal is positioned on an insulating plate which is in direct contact with a liquid medium contained in a radial-symmetrical container and wherein the semiconductor laser is positioned so as to be capable of exposing the liquid medium. The device uses an insulating plate made of a material having an acoustic wave absorption coefficient of no greater than 30 dB/cm and an electromagnetic wave absorption coefficient of no greater than 0.1 Neper. The preferred liquid medium is physiological saline.

The objects are further attained by a device for causing an affect on a biological object, this device according to the invention comprising an exposure source in the form of a crystal comprising at least one semiconductor junction in the structure whereof the changes induced by the action of acoustic and electromagnetic fields of the biological medium are captured; a reproduction pulse generator the outlet whereof is connected in straight polarity to the semiconductor junction of the crystal which is positioned on one face of an insulating cartridge filled with an aqueous medium and another face is in direct contact with the biological object. The device may further comprise a light-emitting diode which is connected in series to the crystal so as it is capable of exposing the aqueous medium. The device uses an insulating cartridge made of a material having an acoustic wave absorption coefficient of no greater than 30 dB/cm and an electromagnetic wave absorption coefficient of no greater than 0.1 Neper, for example, radioengineering ceramics or fluoroplastic.

The objects are further attained by A device for causing an affect on a biological object, the device according to the invention comprising an exposure source in the form of a crystal comprising at least one semiconductor junction in the structure whereof the changes induced by the action of acoustic and electromagnetic fields of the biological medium are captured; a reproduction pulse generator the outlet whereof is connected in straight polarity to the semiconductor junction of the crystal the outlet whereof is connected to a light-emitting diode for exposing the biological object. The device may further comprise a flexible probe in the form of a light guide aligned with the light-emitting diode.

The objects are further attained by a variant of the device for causing an affect on a biological object, the device according to the invention comprising an exposure source in the form of a crystal comprising at least one semiconductor junction in the structure whereof the changes induced by the action of acoustic and electromagnetic fields of the biological medium are captured; a reproduction pulse generator the outlet whereof is connected in straight polarity to the semiconductor junction of the crystal the outlet whereof is connected to a semiconductor laser for exposing the biological object. The device may further comprise a flexible probe in the form of a waveguide aligned with the semiconductor laser.

It is a known fact that separate regions of the plasmatic membrane of a living cell or the entire membrane of a living cell are in an excited oscillating state: these are coherent hypersonic oscillations and electric fields of the ultrahigh-frequency range. This was first noticed by the team of Russian researchers headed by N.D. Devyatkov (see N. D. Devyatkov, M. B. Golant, and O. V. Betsky, Millimeter-Range Waves and Their Role in Vital Processes, Moscow: Radio i Svyaz, 1991, p. 3). Slightly later and independently of the Russian scientists, this hypothesis obtained an indirect theoretical support from H. Frohlich (see H. Frohlich, J. Quant. Chem., 1968, Vol. 2, p. 64). There are all grounds to think that the coherent oscillations according to Frohlich and the acoustoelectric oscillations discussed by the Devyatkov's team are the same physical phenomenon. The power of the electromagnetic oscillations excited by electric dipoles of cellular plasmatic membranes is approximately $10^{-23}$ W within a narrow frequency band. Therefore, such a low value of power is a significant value for living cells, so that in compliance with the reciprocity principle, cells "should be" responsive to external fields of the same order of magnitude. Further, know are concerted effects in the behavior of a bacterial colony up to organizing ordered structures of biofilm type (see V. D. Gruzina, Communicative Signals of Bacteria, website@antibiotic.ru). Naturally, the existence of communicative links in such a structure leads to a considerable enhancement of hypersonic and electric fields of these ordered structures compared to a single biological object.

If a crystal is placed in a container containing a biological medium, for example, a bacterial colony in physiological saline, or if direct contact is provided between this medium and a crystal, hypersonic oscillations will be excited in the crystal and electric fields generated by the bacterial colony will appear. Direct contact may be provided by spreading, over the physiological saline surface in a Petri dish, of a thin insulating layer (an insulating plate) having low absorptions of hypersonic waves and high-frequency electric fields. The crystal is located on top the insulating layer. The hypersonic oscillations of the medium acting on the crystal surface lead to the excitation of hypersonic waves in the crystal. With this, acoustoelectronic interactions are known to occur in the crystal (see Acoustoelectronic Interactions (AEI) entry in the Physical Encyclopedic Dictionary, Moscow, Sovetskaya Entsiklopediya, 1984., p. 17; B. M. Milinkas and A. A. Schuka, "Functional Acoustoelectronics," Funktsional. Elektron., 2002, No. 4, pp. 59-62). Acoustoelectric waves are perturbations resulting from crystal distortions. These distortions take place for some atoms and are associated with a change in distance between them. Displacements of lattice atoms caused by a hypersonic wave change intracrystal fields, which affects the distribution and motion of conductivity electrons. In turn, the redistribution of electrons and their directional motion change the strain pattern and the character of propagation of an acoustic wave through the crystal. In other words, the perturbation of the crystal structure represents a unique spatial reflection of acoustic and electromagnetic fields of the bacterial colony.

It is a natural property of semiconductors to capture changes in crystal structure both in natural settings and, with a far greater probability, under external impacts.

Factors inducing structural changes in crystals are known. For example, in manufacturing semiconductor devices (e.g., *p*-n junctions), strictly dosed amounts of donor and acceptor dopants are added (see the Physical Encyclopedic Dictionary, Moscow, Sovetskaya Entsiklopediya, 1984. p. 568). These dopants change the crystal structure and generate holes and relatively free electrons to provide electrical conductivity. Under the action of an electric field ($E \leq 10^5$ V/cm), break-down of the semiconductor can occur (see the Physical Encyclopedic Dictionary, Moscow, Sovetskaya Entsiklopediya, 1984, p. 567) to cause irreversible structural alterations. Thermal break-downs are also possible. For germanium semiconductors, for example, working temperatures do not exceed 90°C; for silicon semiconductors, 120°C. Higher values also cause irreversible (captured) changes in the crystal structure. Noteworthy, the electric field strengths inducing changes in crystal structure are relatively low and may easily be provided by a pulse generator. The aforementioned crystal structure alterations result from extreme external impacts. However, there is a method for diagnosing the longevity of semiconductor devices by fluctuation parameters (flicker noise). Some of these fluctuations cause irreversible changes in crystal structure, and in the absence of external impacts, too (see M. A. Kitaev, A.V. Moryashin, S.V. Obolensky, and A.V. Yakimov, Manifestation of Natural Degradation of Schottky Field-Effect Transistors in Current-Voltage and 1/f Noise, in Proceedings of XXXV International Scientific and Methodological Workshop "Noise and Degradation in Semiconductor Devices," MNTORES im. A. S. Popov, 2006, pp. 39-44).

For crystals having piezoelectric properties, acoustic perturbations in a crystal can be considerably enhanced under a sufficiently strong electric field. The integrated intensity can reach high values so that to change the macroscopic properties of the crystal. As a rule, electrical conductance decreases in response; a considerable part of the voltage applied to the crystal affects regions having higher acoustic phonon intensities. In this way, an instability appears and generates regions of a strong electric field and a high acoustic phonon intensity, what is called acoustoelectric domains. Static domains immovably frozen in the crystal structure may be observed experimentally (see the Acoustoelectronic Interactions entry in the Physical Encyclopedic Dictionary, Moscow, Sovetskaya Entsiklopediya, 1984, p. 17).

Thus, for the changed macroscopic properties of a crystal to be captured, a sufficiently strong electric field should be applied to the crystal. In the method for capturing changes in crystal structure induced by the acoustic and electromagnetic fields of a biological medium, the fields is applied to the semiconductor junction formed on the crystal surface. The field is excited by the capture pulse of a pulse generator. For a single pulse impact, the total heating up of the crystal is 0.003°C, while the local heating up of the junction area does not exceed 3°C. This temperature change does not induce any additional structural changes in the crystal.

In the course of experiments, the authors discovered that the changes induced by acoustic and electromagnetic fields are captured on the potential barrier of the crystal only when a sufficiently high-voltage opposite-polarity pulse having a duration of no longer than 1 second is applied. When the semiconductor junction of the crystal is impacted with a cut-off polarity pulse, an electric field of a high strength ($E = 10^5$ V/m) is generated in the junction area, leading to a considerable enhancement of acoustic perturbations in the crystal and generating acoustoelectric domains.

The fact that, in the moment of capture, the crystal is positioned on an insulating plate which is in contact with a biological medium on account of the surface tension of the medium placed in a container, allows conductive contact between the crystal and the medium to be avoided without losing the capture quality and, thereby, possible distortions from direct contact to be ruled out. The insulating plate is preferably made of a material having an acoustic wave absorption coefficient of no greater than 30 dB/cm and an electromagnetic wave absorption coefficient of no greater than 0.1 Neper, for example, radioengineering ceramics of fluoroplastic.

The changes induced by the action of acoustic and electromagnetic fields of a biological medium which is under the impact of an inactivating agent are captured in a similar manner. The inactivating agent may be any disinfectant, for example, Lysoformin, as well as temperature, UV radiation, and osmotic shock. The distinction of this method consists in that the excitation of a crystal by the action of a cut-off polarity electric pulse on the semiconductor junction of the crystal occurs at the time of the peak level of the field of the biological medium as determined experimentally for the type of biological medium used under the impact of the inactivating agent. The cut-off polarity electric pulse may be a square pulse having amplitude of 3 to 4 V and duration of 0.1 to I second.

The initial capture of the structural changes in crystalline elements is a rather laborious and expensive procedure requiring participation of highly skilled persons. In handling with biological media, such as AIDS viruses or stem cells, this is feasible only at special research institutions. Therefore, the invention solves the second problem, namely, creates a method for duplicating the structural changes that occurred in an initial crystal in the structure of a duplicate crystal.

The method for duplicating the changes that occurred in an initial crystal structure in a duplicate crystal structure according to a first variant of the invention is characterized in that it uses crystals the structure whereof comprises at least one semiconductor junction, wherein the changes induced by the acoustic and electromagnetic fields of a biological medium are captured in the structure of one crystal and wherein both crystals are positioned on one face of a water-filled hollow insulating cartridge.

Water is known to have unusual properties (see O. V. Betsky, N. N. Lebedeva, and T. I. Kotrovskaya, Unusual Properties of Water in Weak Electromagnetic Fields, Biomed. Tekhnol. Radioelektron., 2003, No. 1, pp. 37-43). When a reproduction straight-polarity pulse passes through the semiconductor junction of a crystal wherein the changes induced by the action of acoustic and electromagnetic fields of a biological medium are captured, reproduction and subsequent action of these fields on the aqueous medium occur. In several minutes, rather a local impact on the aqueous medium induces structural alterations in rather a large volume of the aqueous medium. The aqueous medium serves as an accumulator of the acoustic and electromagnetic fields wherein the second crystal occurs. The semiconductor junction of the initial crystal is repeatedly impacted by a reproduction straight-polarity pulse, after which the semiconductor junction of the duplicate crystal is impacted by a cut-off polarity capture pulse. When the cut-off polarity capture pulse passes through the semiconductor junction of the duplicate crystal, the duplicate crystal captures the changes induced by the acoustic and electromagnetic fields in the aqueous medium, which are identical to the acoustic and electromagnetic fields of the biological medium that has induced changes captured by the initial crystal structure. The processes involved here are identical to the processes involved in capturing the changes induced by the action of acoustic and electromagnetic fields of the biological medium in the initial crystal.

A second variant of the method for duplicating the changes occurring in an initial crystal structure in a duplicate crystal structure is characterized in that the duplicate crystal is positioned on an insulating plate which is in direct contact with a liquid medium contained in a radial-symmetric container, wherein the semiconductor junction of the initial crystal is repeatedly impacted by a straight-polarity reproduction pulse; the generated electric current oscillations modulate the light flux of a powerful (1-100 mW) semiconductor laser to expose the liquid medium, after which the semiconductor junction of the duplicate crystal is impacted by a cut-off polarity capture pulse. The preferred liquid medium is physiological saline.

Under the action of the modulated light flux, a hypersonic standing wave is excited in the liquid medium. The fact that the liquid medium is contained in a radial-symmetric container allows creating a region of maximal hypersonic wave amplitudes and positioning the duplicate crystal within this region on an insulating plate which is in direct contact with the liquid medium. Capturing is performed by sending a capture cut-off polarity pulse to the semiconductor junction of the duplicate crystal. When the cut-off polarity capture pulse passes through the semiconductor junction of the duplicate crystal, the duplicate crystal captures the changes induced by the acoustic and electromagnetic fields in the aqueous medium, which are identical to the acoustic and electromagnetic fields of the biological medium that has induced the changes captured by the initial crystal structure. The physics of the capturing processes in this method and in all others (including capture by initial crystals) is the same.

The method for causing an effect on a biological object wherein the exposure source is a device comprising a crystal the structure whereof comprises at least one semiconductor junction, with the changes induced by the acoustic and electromagnetic fields of the biological medium being captured by the crystal structure, may be carried out by variants of the invention. In a first variant of the invention, the method is characterized in that the crystal is positioned on one face of a water-filled hollow insulating cartridge which has its another face in direct contact with the biological object, and the semiconductor junction of the crystal is repeatedly exposed to a straight-polarity electric pulse.

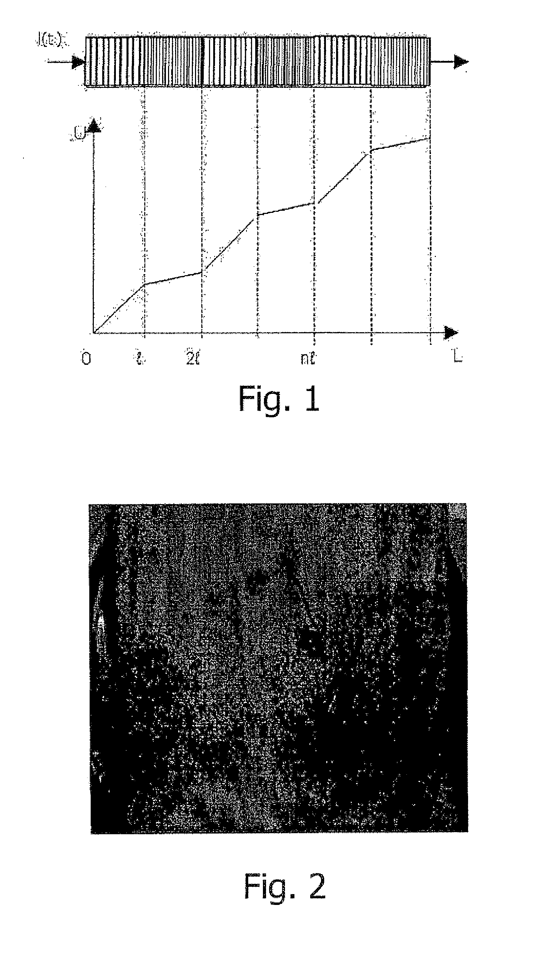

A *p-n* junction of a crystal originally has well-defined inhomogeneity (see Fig. 1). When the crystal structure captures the changes induced by the action of acoustic and electromagnetic fields of the biological medium, the character of inhomogeneity changes. The potential distribution along an inhomogeneous structure may be represented as a sequence of dipole structures superimposed on a homogeneous material. In turn, each dipole structure can serve as an antenna for exciting at least a quasi-static electric field in the surrounding medium (see the Physical Encyclopedic Dictionary, Moscow, Sovetskaya Entsiklopediya, 1984, p. 25).

The characteristic inhomogeneity scale is defined by the relationship

$\ell = c/\pi\omega$, where c is light velocity and $\omega$ is circular frequency of electromagnetic radiation.

With this, in the exciting pulse spectrum $I(t)$

$$S_1(\omega) = $$

$$\int_0^T I(t)e^{i\omega t}\,dt$$

where *T* is exciting pulse period and *i* is an imaginary unit, the spectral component of frequency ω should be powerful enough. Under these conditions, an inhomogeneous structure is an enough efficient antenna for emitting electromagnetic waves of frequency ω under the action of the pulse *I*(*t*).

When a straight-polarity reproduction pulse acts on the junction of a crystal., an inhomogeneous electric field appears in the crystal as a result of the inhomogeneity thereof. Accordingly, an electromagnetic field appears in the surrounding medium (at least, in the nearest lying zone) to repeat, in the spatial and temporal parameters thereof, the field of the biological medium; as a result, acoustic and electromagnetic fields are generated in the aqueous medium. The action of this field on a biological object gives the desired therapeutic effect. When a man or an animal is intended to be exposed, the cartridge is positioned in the area of projection of a pathologic organ and repeated exposure is performed. The cartridge with the aqueous medium considerably increases the area of the patient's skin in the correcting field.

In a second variant, the method for causing an affect on a biological object is characterized in that the semiconductor junction of the crystal is repeatedly exposed to a straight-polarity reproduction pulse, the thus-generated oscillations modulate the light flux of a light-emitting diode or a semiconductor laser directed to the skin of a man of an animal in the area of projection of a pathologic organ or to the surface of a hollow organ, and exposure is carried out in a schedule of two to six sessions a day, each lasting 10 to 60 second, for at least five days.

The use of high-frequency modulation of the light flux for healing and regenerating tissues is known (see the Russian Federation patent No. 2314849-, publ. 2007). The method consists of exposing skin regions to terahertz radiation carried by infrared radiation. The carrier radiation serves to provide an enhanced penetration depth of terahertz frequencies into biological tissues, thereby increasing the efficiency of photo-stimulated biochemical reactions on account of the coincidence of the terahertz radiation frequency with the resonance oscillation frequencies of intercellular (and intracellular) membranes and protein molecules. However, the modulation law in this method is wide-band, and only an insignificant part of the absorbed radiation energy is transmitted to narrow resonance oscillations.

In the method according to the second variant of the invention, the light flux of a light-emitting diode or a semiconductor laser is modulated by the electric current oscillations generated by passage of a straight-polarity reproduction pulse on account of the inhomogeneity of a crystal the structure whereof captures the changes induced by the action of acoustic and electromagnetic fields of a biological medium. Herein, modulation frequencies are specific for each biological object and are the intrinsic frequencies of this object. Thereby provided is the high efficiency of the interactions between the radiation and the biological object.

When ill body organs are hollow, a flexible probe may be useful in the form of a light guide aligned with the light-emitting diode or semiconductor laser.

Brief Description of Drawings

Further, the invention will be explained with reference to the drawings attached.

Figure 1 shows a unit having structural inhomogeneities and the electric pulse potential distribution along its length U(L);

Figure 2 shows the positioning of the initial crystal on the surface of an insulating plate while capturing the structural changes thereof induced by the acoustic and electromagnetic fields of a biological medium.

Figure 3 represents a device for capturing the changes in the initial crystal structure induced by the acoustic and electromagnetic fields of a biological medium.

Figure 4 represents a device for duplicating the changes in the initial crystal structure in the duplicate crystal structure.

Figure 5 represents a variant of a device for duplicating the changes in the initial crystal structure in the duplicate crystal structure.

Figure 6 represents a device for causing an affect on a biological object.

Figure 7 shows "inhibited bacterial growth" zones around crystals wherein the changes in the initial crystal structure induced by the action of acoustic and electromagnetic fields of a biological medium are captured in the crystal structure.

Figure 8 shows the absence of growth inhibition on crystals having unchanged structure.

Figure 9 represents a variant of a device for causing an affect on a biological object.

Figure 10 represents a variant of a device for causing an affect on a biological object.

Figure 11 represents a variant of a device for causing an affect on a biological object.

Figure 12 represents a variant of a device for causing an affect on a biological object.

Figure 13 represents a variant of a device for causing an affect on a biological object. Embodiment Examples of the Invention

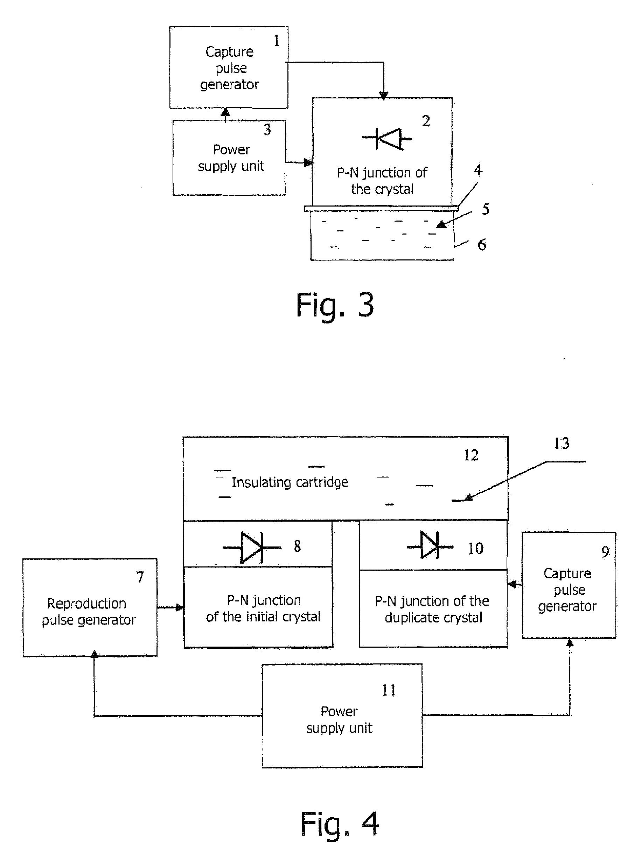

Example 1. A method for capturing the changes induced by the action of acoustic and electromagnetic fields of a biological medium and a device for carrying out the method (Figs. 2, 3).

The device (see Fig. 3) comprises a capture pulse generator 1 the outlet whereof is connected in cut-off polarity to the semiconductor junction of a crystal 2 and a power supply unit 3. The crystal 2 is mounted on an insulating plate 4 which is in contact with a biological medium 5 placed in a container 6. Figure 2 shows the position of the crystal during capturing. The insulating plate 4 is made of a material having low absorption of hypersonic waves and high-frequency electric fields, for example, of radioengineering ceramics or fluoroplastic. The pulse generator 1 may be designed as a mono-oscillator which generates a triangular or square electric pulse having duration of 0.1 to 1 sec and amplitude of 3 to 4 V. In the present invention, the generator is implemented on the basis of an Atmel AT 90S1200-4SI microcontroller. This chip is now best for use in cost/quality terms. The microcontroller is programmed to generate an output square pulse.

The method for capturing the changes induced in a crystal by the action of acoustic and electromagnetic fields of a biological medium is carried out as follows. In the crystal 2 positioned on the insulating plate 4, excited are hypersonic oscillations and electric fields generated by the biological medium 5. As a result, distortions appear in the crystal. The perturbation of the crystal structure represents a unique spatial reflection of the acoustic and electromagnetic fields of the biological medium 5. When the semiconductor junction of the crystal 2 is exposed to the action of a cut-off polarity pulse of the capture pulse generator 1, a high-strength electric field ($E = 10^5$ V/cm) appears in the region of the junction, which considerably enhances perturbations in the crystals and gives rise to acoustoelectric domains. When the capture pulse ends, the structure of the crystal 2 captures changes induced by the action of acoustic and electromagnetic fields of the biological medium 5.

The changes induced by the action of acoustic and electromagnetic fields in the crystal structure may be captured by exposure to any medium, for example, various microorganism cultures in an inactivated (suppressed) condition, medicaments, quality antibodies of blood serum, and others.

The changes induced by the action of acoustic and electromagnetic fields of a biological medium which is under the impact of an inactivating agent are captured in the crystal structure in a similar manner. The inactivating agent may be any disinfectant, for example, Lysoformin, as well as temperature, UV radiation, and osmotic shock. The distinction of this capturing method consists in that the excitation of the semiconductor junction of the crystal 2 by a cut-off polarity electric pulse occurs at the time of the peak level of the field of the biological medium as determined experimentally for the types of biological medium and inactivating agent used. Experiments discovered that for the exposure of a biological medium to an inactivating agent, the field characteristic is a one-humped curve. The time in which a peak field will be observed depends on the type of biological medium and the type of inactivating agent. For S. Aureus microorganisms and for Lysoformin disinfectant, a peak field (a high capture level) was observed in 4 minutes after Lysoformin was applied, whereas for E coli, a peak field was observed at the 10the second after Lysoformin was applied.

Example 2. A method for duplicating the changes that occurred in an initial crystal structure in a duplicate crystal structure and a device for carrying out the method (see Fig. 4).

The device comprises a reproduction pulse generator 7 which is connected in straight polarity to the semiconductor junction of an initial crystal 8 and a capture pulse generator 9 which is connected in cut-off polarity to the semiconductor junction of a duplicate crystal 10. The reproduction pulse generator 7 and the capture pulse generator 9 are connected to a power supply unit 11. The crystals 8 and 10 are positioned on one broad face of a hollow insulating cartridge 12 which is filled with an aqueous medium 13. As the crystal 8, used is an initial crystal whose structure has captured the changes induced by the action of acoustic and electromagnetic fields of the biological medium by a method according to the method described in Example 1.

The method is carried out as follows. Feeding signals from the reproduction pulse generator activates the initial crystal 8; a straight polarity pulse through the junction of the crystal 8 in whose structure the changes induced by the action of acoustic and electromagnetic fields of a biological medium are captured, generates identical fields in the aqueous medium 1.3, and these fields act on the crystal 10. A cut-off polarity pulse from the capture pulse generator 9 fed to the junction of the crystal 10 changes its structure in accordance with the structure of the initial crystal 8, wherein the structural changes occurred in response to the action of the fields of the biological medium. The processes involved here are identical to the processes involved in capturing the changes, induced by the action of acoustic and electromagnetic fields of the biological medium, in the structure of the semiconductor junction of the crystal (see Example 1). This device provides an easy duplication of the structural changes in crystals in whose structure the changes induced by the action of acoustic and electromagnetic fields of any biological medium are captured.

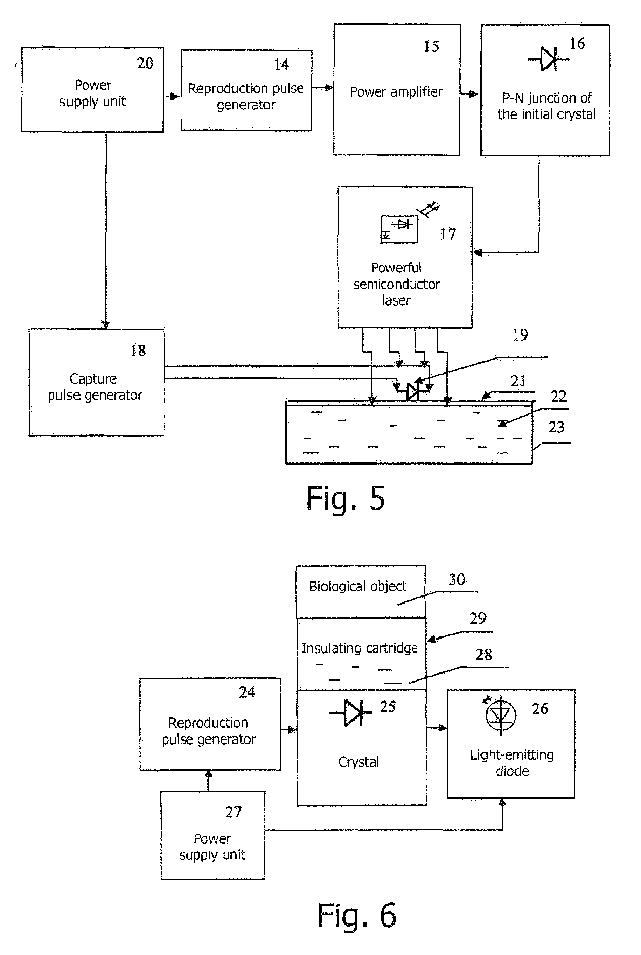

Example 3. A variant of a method for duplicating the changes that occurred in an initial crystal structure in a duplicate crystal structure and a device for carrying out the method (see Fig. 5).

The device comprises a reproduction pulse generator 14 which is connected in straight polarity through a power amplifier 15 to the semiconductor junction of an initial crystal 16, the outlet whereof is connected to a powerful semiconductor laser 17, and a capture pulse generator 18, which is connected in cut-off polarity to the semiconductor junction of a duplicate of the initial crystal 19. The reproduction pulse generator 14 and the capture pulse generator 18 are connected to a power supply unit 20.

The duplicate crystal 19 is positioned on an insulating plate 21 which is in direct contact with a liquid medium 22 placed in a radial-symmetric container 23, and the semiconductor laser 17 is positioned so that it can expose the liquid medium 22. The device uses a semiconductor laser having a power of 10 to 100 mW. As the crystal 18, used is the initial crystal in whose structure the changes induced by the action of acoustic and electromagnetic fields of a biological medium are captured according to the method described in Example 1.

The method is carried out as follows.

Signals from the reproduction pulse generator 14 are amplified by the power amplifier 15 and excite the initial crystal 16. As a result of the inhomogeneity of the crystal 16, an inhomogeneous electric field appears in it and, as a consequence, electric current oscillations. The electric current oscillations modulate the light flux of the powerful semiconductor laser 17, which acts on the liquid medium 22 placed in the radial-symmetric container 23 (for example, a Petri dish). When the liquid medium 22 is exposed to the modulated radiation of the semiconductor laser 17, a hypersonic wave is excited in the medium, and the duplicate crystal 19 is positioned precisely in the peak wave amplitude region. The semiconductor junction of the duplicate crystal 19 is exposed to the action of a cut-off capture pulse. When the cut-off capture pulse passes through the semiconductor junction of the duplicate crystal 19, the duplicate crystal captures the changes induced by acoustic and electromagnetic fields of the aqueous medium 22 which are identical to the acoustic and electromagnetic fields of the biological medium that induced the changes captured in the initial crystal structure (see Example 1). These fields are identical and have a complex (electric and hypersonic) character.

This device provides an easy duplication of the structural changes in crystals in whose structure the changes induced by the action of acoustic and electromagnetic fields of any biological medium are captured.

Example 4. A method for causing an affect on a biological object and a device for carrying out the method (see Fig. 6).

The device comprises a reproduction pulse generator 24 which is connected in straight polarity to the semiconductor junction of a crystal 25 connected in series to a light-emitting diode 26, and a power supply unit 27, which is connected to the reproduction pulse generator 24 and the light-emitting diode 26. The crystal 25 is positioned on one broad face of a hollow insulating cartridge 28 which is filled with an aqueous medium 29. The light-emitting diode 26 is positioned so that it can expose the aqueous medium 29. As the crystal 25, used is a crystal in whose structure the changes induced by the action of acoustic and electromagnetic fields of a biological medium are captured according to the method described in Examples 1,2, and 3.

The method for exposing a biological object is carried out as follows. The insulating cartridge 29 is positioned so that its free broad face lies directly on the surface of a biological object 30. The biological object may be any biological medium, or a man, or an animal. Feeding signals from the reproduction pulse generator 24 activates the crystal 25; straight polarity pulses passing through the junction of the crystal 25 generate acoustic and electromagnetic fields in the aqueous medium 29 to act on the biological object 30. Depending on the type of field inducing the changes captured in the structure of the crystal 25 (inactive condition of a microorganism, a medicament diluted in water, quality antibodies of blood serum, and others), the biological object 30 changes the properties thereof in an appropriate manner under the action of this field. The light-emitting diode 26 connected in series to the crystal 25 emits a light flux which is amplitude-modulated by the electric current oscillations that appear upon the excitation of electromagnetic fields in the crystal 25, and this flux acts on the aqueous medium 29. As a result, apart from the aforementioned electromagnetic fields, the properties of the biological medium 5 are reproduced by the modulated emission of the light-emitting diode 26.

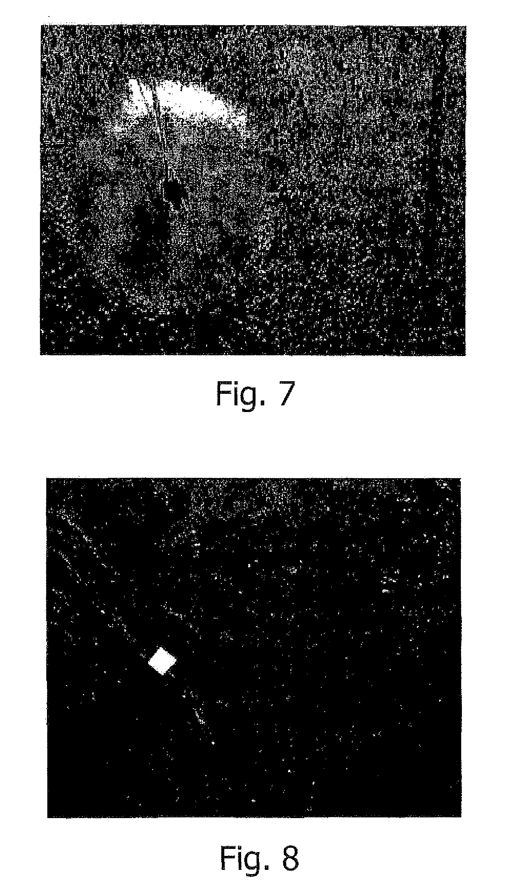

The efficiency of capturing the field of a biological medium and the efficiency of exposure were evaluated as the size of "inhibited bacterial growth" zones under the direct action of the crystal. The experiment was carried out as follows. A nutritive gel was placed in a Petri dish. The gel surface was uniformly inoculated with a bacterial dispersion in physiological saline. A crystal whose structure had captured the changes induced by the action of acoustic and electromagnetic fields of a colony of the same bacteria after being exposed to the action of Lysoform disinfectant was placed directly in the culture (see Fig. 7); in another portion of the dish, a placebo crystal having unchanged structure was placed for reference (see Fig. 8). Both crystals were exposed to the action of an electric pulse, after which the Petri dish containing the bacterium-inoculated nutritive gel and the crystals was covered with a cover and transferred to a thermostat at T=37°C. After one day elapsed, the following pattern was observed. Figure 7 shows "inhibited bacterial growth" zones around the crystals whose structure had captured the changes induced by the action of acoustic and electromagnetic fields of the biological medium, proving the efficiency of capturing and subsequent exposure. Around the crystals having unchanged structure, no growth inhibition zones were seen (Fig. 8).

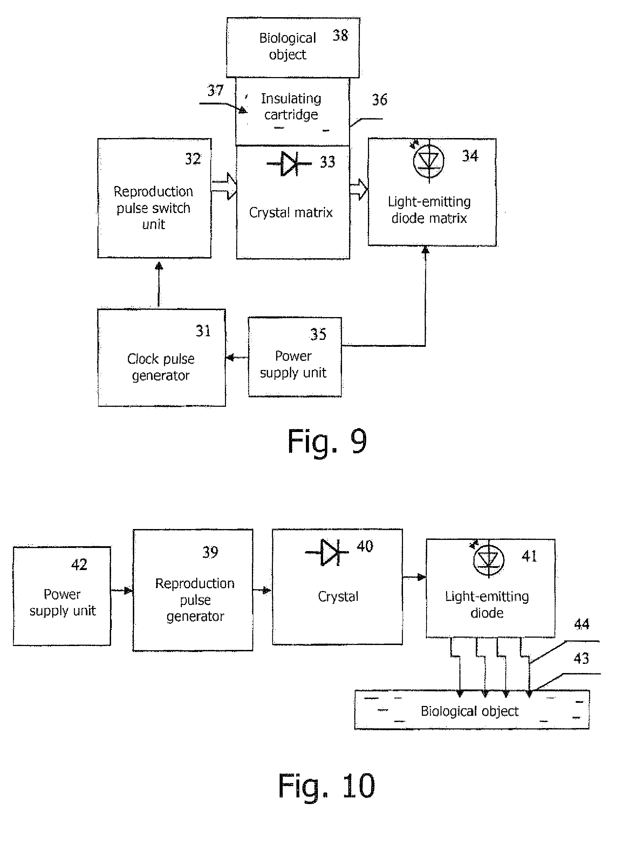

Example 5. A method for causing an affect on a biological object wherein the source of exposure is a matrix of semiconductor junctions whose structure has captured the changes induced by the action of acoustic and electromagnetic fields of a biological medium and a device for carrying out the method (see Fig. 9).

The device comprises a clock pulse generator 31 the outlet whereof is connected to the reproduction pulse switch unit 32; a crystal matrix 33, each crystal containing at least one semiconductor junction connected in series to a light-emitting diode of a light-emitting diode matrix 34; and a power supply unit 35. The matrix 33 is positioned on one broad face of a hollow insulating cartridge 36 which is filled with an aqueous medium 37. The light-emitting diode matrix 34 is positioned so that it can expose the aqueous medium 37. The outlets of the switch unit 32 are connected in straight polarity to the semiconductor junctions of the matrix 33. The pulse switch unit is designed on the basis of decade counters with decoders of output states (for example, K56111E8) which are well known (see A. L. Lantsov, L. N. Zvorykin, and I. F. Osipov, Digital Devices on Complementary MIS Integrated Circuits, Radio i Svyaz, 1983, pp. 60-62). As the crystals in the matrix 33 used are crystals whose structure has captured the changes induced by the action of acoustic and electromagnetic fields of one or more biological media according to the method described in Examples 1, 2, and 3.

The method for causing an affect on a biological object is carried out in the following manner.

The insulating cartridge 36 is positioned with the free broad face thereof lying immediately on the surface of a biological object 38. The biological object may be any biological medium placed in a container, or a man, or an animal. Feeding signals from the clock pulse generator 31 activates the reproduction pulse switch 32, from which pulses are consecutively fed to each semiconductor junction of the matrix 33, wherein a straight polarity pulse through one of the junctions of the matrix 33 excites, in the aqueous medium 37, acoustic and electromagnetic fields that act on the biological object 38. Changes in structure induced by either one and the same field or the field of different biological media may be captured on the semiconductor junctions of the matrix 33. Depending on the field type inducing the structural changes in a given junction of the matrix 33 (an inactive condition of a microorganism, water-diluted drug, quality serum antibodies, and others), the biological object 38 changes its properties under the action of this field. A light-emitting diode of the matrix 34 connected in series to a crystal of the matrix 34 emits a light flux which is amplitude-modulated by electric current variations appearing upon the excitation of electromagnetic fields in a crystal of the matrix 37. The light flux emitted by light-emitting diodes of the matrix 34 acts on the liquid medium 37. As a result, apart from the aforementioned electromagnetic fields, the fields of the biological medium are reproduced by the modulated emission of a light-emitting diode of the matrix 34.

Example 6. A variant of the method for causing an affect on a biological object and a device for carrying out the method (see Fig. 10).

The device comprises a reproduction pulse generator 39 connected in straight polarity to the semiconductor junction of a crystal 40, which is connected in series to a light-emitting diode 41, and a power supply unit 42, which is connected to the reproduction pulse generator 39. As the crystal 40 used is a crystal whose structure has captured the changes induced by the action of acoustic and electromagnetic fields of a biological medium according to the method described in Examples 1 to 3.

The method for causing an affect on a biological object is carried out in the following manner. The device is positioned so that the light flux from the light-emitting diode 41 exposes a biological object 43. The biological object may be any biological medium placed in a container, or a man, or an animal. Feeding signals from the reproduction pulse generator 39 generates an inhomogeneous electric field in the crystal 40, which in turn generates an inhomogeneous electric field in the surrounding medium; provided that known relationships are held between the oscillation spectrum of an $I(t)$ exciting pulse and the spatial inhomogeneity scale $\ell$ (see Fig. 1), rather an efficient excitation of electromagnetic waves can occur. The electric current oscillations generated in this way amplitude-modulate the light flux 44 of the light-emitting diode 41. As a result, the fields excited in the crystal 40 (which are the reflection of the acoustic and electromagnetic fields of the biological medium 5) are reproduced by the modulated emission of the light-emitting diode 41.

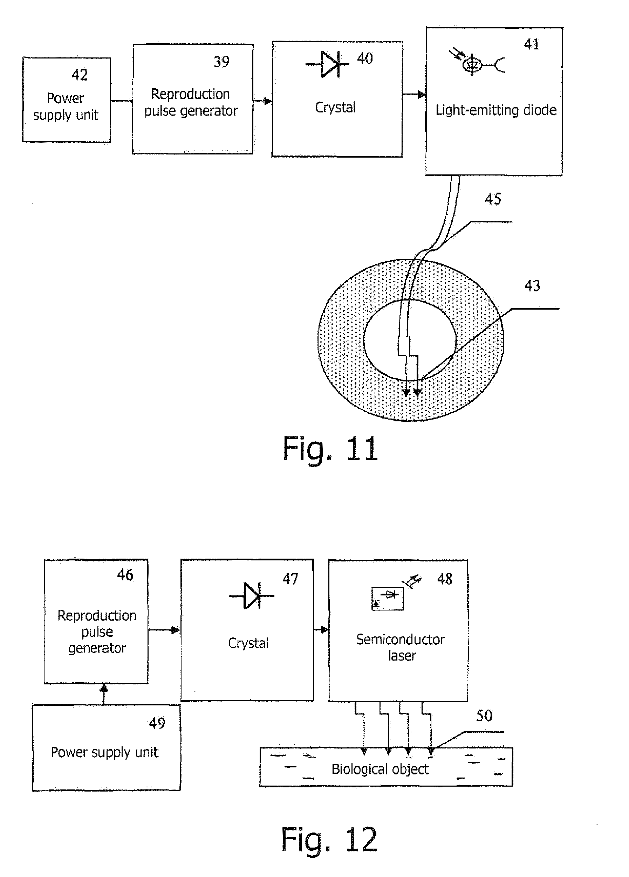

Example 7. A variant of the device for causing an affect on a biological object (Fig. 11).

The distinction of the device in Fig. 11 from the device shown in Fig. 10 consists of a flexible probe 45 in the form of a light guide aligned with the light-emitting diode 41. This assembly is intended for exposing the inner surface of a hollow organ of a man or an animal. The flexible light guide 45 is entered a hollow organ of the biological object 43 to be located opposite the focus of pathology. Further, the method is carried out as described in Example 6.

Example 8. A variant of the method for causing an affect on a biological object and a device for carrying out the method (see Fig. 12).

The device comprises a reproduction pulse generator 46 connected in straight polarity to the semiconductor junction of a crystal 47, which is connected in series to a semiconductor laser 48, and a power supply unit 49, which is connected to the reproduction pulse generator 46. As the crystal 47 used is a crystal whose structure has captured the changes induced by the action of acoustic and electromagnetic fields of a biological medium according to the method described in Examples 1, 2, and 3.

The method for causing an affect on a biological object is carried out in the following manner. The device is positioned so that the light flux from the semiconductor laser 48 exposes a biological object 50. The biological object may be any biological medium placed in a container, or a man, or an animal. Feeding signals from the reproduction pulse generator 46 excites an electric current in the crystal 47. The electric current oscillations generated in the inhomogeneous crystal amplitude-modulate the light flux from the semiconductor laser 48. As a result, the acoustic and electromagnetic fields of the biological medium 5 which induced changes in the crystal 47, are reproduced by the modulated emission of the semiconductor laser 48.

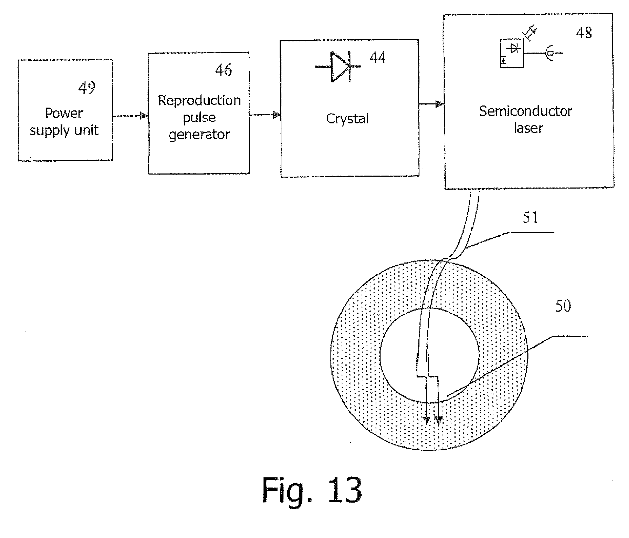

Example 9. A variant of the device for causing an affect on a biological object (see Fig. 13).

The distinction of the device in Fig. 13 from the device shown in Fig. 12 consists of a flexible probe 51 in the form of a light guide aligned with the light-emitting diode 48. The flexible light guide 51 is entered a hollow organ of the biological object 50 to be located opposite the focus of pathology. Further, the method is carried out as described in Example 8.

For validating the efficiency of the device for causing a therapeutic effect (hereinafter, an instrument), the biocidal activity thereof was studied against test cultures of microorganisms: S. aureus (collection (blank) strain No. 906 and E. coli. (collection (blank) strain No. V-8208), both purchased from GISC named after Tarasevich. Assay bacterial dispersions were rapidly inoculated on nutrient medium kits (meat-peptone agar (MPA), meat-peptone broth (MPB), Endo medium, and sugar broth) in equal volumes of 0.1 ml each. Incubation was carried out in a thermostat at $37\pm0.3°C$ for 18 to 24 hours. What was recorded was the occurrence of a visual microbian growth with the description of growth characteristics, careful counting of grown colonies, and microscopic examination of smears with Gram staining and with the determination of their phenotype and genotype characteristics, as well as cultural and biochemical properties and the resistance profiles of microorganisms. Simultaneously with the assay bacterial dispersions, blank dispersions were prepared in a similar manner from the same microorganisms; these blank dispersions were not exposed to the action of a device but were also kept in a thermostat at $37\pm0.3°C$. For the blanks, sampling and inoculation were performer under the same conditions and at the same time as for the assay samples.

Example 10. The effect of an instrument comprising a crystal whose structure has captured the changes induced by the action of acoustic and electromagnetic fields of the biological medium of S. aureus in an inactive (suppressed) condition on an identical culture in an active condition. The operation schedule of the instrument was as follows: 10-second exposures twice a day for 3 days.

Biocidal properties. After S. aureus in physiological saline was exposed to the action of the instrument, a 95% decrease in CFU was noticed in the 3rd day; single S. aureus colonies (two or less) were inoculated. In an experiment carried out on a meat-peptone broth with the continuous daily monitoring of inoculations, the complete absence of S. aureus growth was detected. In the S. aureus blank on physiological solution and S. aureus on MPB, continuous growth was noticed in all assays.

Biochemical properties. Prior to the exposure to the instrument, in the course of the experiment, and after it was over, as well as in the blank, the identification of S. aureus was carried out in full. Biochemical properties were carried out in three replicate studies. No changes in biochemical properties were detected after exposure to the action of the instrument.

Sensitivity to antibiotics. Prior to the experiment, a resistivity profile was determined for the collection S. aureus strain. Sensitivity was revealed to Oxacyclin, Cephalothin, Cephazolin, Cefotaxim, Cephalexin, Cefuroxim, Kanamycin, Gentamycin, Erythromycin, Lincomycin, Clindamycin, Rifampin, Ciprofloxacin, and Vancomycin; resistance was revealed to benzylpenicillin. After the assays, the resistance profile did not change.

Example 11. Exposure to an instrument comprising a crystal whose structure has captured the changes induced by the action of acoustic and electromagnetic fields of a biological medium, which was an inactive condition of coli bacillus and Gentamycin antibiotic on E. coli M-17. The operation schedule of the instrument was a follows: $2 \times 10$ seconds a day for 3 days.

The results were evaluated in 72 h. As a result of exposure to the instrument, growth reduction to single colonies in physiological saline and the absence of growth in a meat-peptone broth were observed in the assay. Thus, the instrument causes a biocidal effect on E. coli Me17.

Morphological changes. When an E. coli M-17 culture was viewed with a microscope, the appearance of coli bacilli changed: their bodies thickened, and they were clustered. In the first day, well-defined morphological alterations were observed: MPB smears showed swollen barrel-like rods with pointed ends; short, thick rods with the ends chopped off were also encountered. Single long thin rods with even edges; doubled rods. The color remained unchanged. Debris, damaged cells, and fragments of dead bacteria with polychromasy were well distinguished on the background of altered living bacteria. In the experiment carried out on physiological saline, Gram-negative living colon bacteria were short; cavities in cells and separate debris of dead cells were seen. In the blanks that were not exposed to action of the instrument both in MPB and physiological saline, E. coli M-17 bacteria were Gram-negative: the bacteria were arranged at an angle and in clusters, swollen as a barrels with pointed ends wherein cavities and vacuoles were seen; some were in the form of double bacilli. In smears taken before the experiments, E. coli M-17 are Gram-negative; rods were short and thin, with rounded ends, and were arranged chaotically.

Biochemical properties. Analysis of biochemical properties did not reveal changes induced by the exposure to the instrument.

Sensitivity to antibiotics. Prior to an experiment, a resistance profile was determined for E. coli M-17 strain. The culture was sensitive to: Ampicillin, Carbenicillin (25 µg), Cefazolin, Cephalothin, Cefuroxim, Cefotaxim, Ceftriaxone, Ceftazidime, Cephalexin, Levomycetin, Kanamycin, Gentamycin, Amikacin, Methylmycin, Streptomycin, Polymyxin, Furadonine, Ofloxacin, and Ciprofloxacin. Low sensitive to tetracycline.

No changes in resistance profile were observed after testing the instrument.

Example 12. Evaluation of the effect of an instrument comprising a crystal whose structure has captured the changes induced by the action of acoustic and electromagnetic fields of an intact liver (hereinafter, the instrument BIK-

EP 2 359 900 A2

NP) in a normal condition on the course of Paracetamol-induced toxic hepatosis in rat.

The experiment was carried out at the Research Center for Toxicology and Hygienic Regulations of Biomedicinals (the town of Serpukhov, Russia).

The study was carried out on 21 pedigree white male rats purchased from the nursery at the Andreevka Branch of the Research Center for Toxicology and Hygienic Regulations of Biomedicinals. Toxic hepatosis in rats was induced by a double administration of water-dispersed Paracetamol. Paracetamol was administered intragastrically by means of a syringe and a bead-ended metallic probe in the following schedule and doses: the first administration, 1000 mg/kg body weight after 6-h starvation; the second administration, 500 mg/kg body weight after 6-h starvation.

The treatment of the animals with the BIK NP instrument was started 42 h after Paracetamol was administered for the first time. The treatment was performed as follows: the radiator of the instrument was applied to the abdominal wall of the animal to be treated in the area of projection of the liver and the "on" button, of the instrument was pressed in several second intervals. One button pressing induced a radiation pulse lasting 1 second. One treatment session of each animal comprised 10 consecutive pulses. The experimental animals were divided into four groups, seven individuals per group. The treatment schedule was designed as shown in. Table 1 below.

Table 1. Experimental design schedule

| Group No | Ind. animal No | Paracetamol administration | Treatment type (instrument) | Session duration (button pressings) | Sessions per day | Session onset time (hour:min) |
|---|---|---|---|---|---|---|
| 1 | 1-7 | Yes | Instrument | 10 | 1 | 09:30; |
| 2 | 8-14 | Yes | Phantom | 10 | 6 | 9:30; 10:30; 11:30; 13:30; 14:30; 15:30 |
| 3 (intact blank) | 15-21 | No | No | - | - | - |

The treatment of animals with the instrument lasted 8 days.

Two blanks were used in the experiment. A positive blank was Group 2, which consisted of rats that received Paracetamol, but instead of the real instrument, a phantom instrument was applied to the abdominal wall. An intact blank was Group 3, which consisted of animals that were not subjected to any manipulations.

In the evening of the 8th treatment day, all animals were deprived of feed but had free access to water. Next day in the morning, blood was sampled from all rats with Medina anesthetization, after which all rats were killed by cervical dislocation and subjected to postmortem autopsy. Necropsy consisted of the investigation of the appearance of animal corpses, as well as organs of the thoracic, abdominal, and pelvic cavities. The corpse and liver weights were determined to be further used for calculating weight coefficients; liver chips were placed in buffered 10% formalin solution for fixing. Blood serum was studied with respect to four indices: alanine amino transferase (ALT), aspartate amino transferase (AST), bilirubin, and alkaline phosphatase. Fixed liver chips were subjected to a standard treatment to obtain paraffin sections 5 $\mu$m thick, which were stained with Hematoxylin and Eosin and then examined with a light microscope. Statistic analysis was carried out using Microsoft Excel and STATISTICA 6.0 software.

Having regard to the fact that the Paracetamol target organ is the liver, the data of the experiment on the background of normal feed intake show a disordered protein-forrning function of the liver, that is, hepatocellular damage of the liver.

Upon the inspection of the biochemical indices of blood serum, statistically significant differences were found for the ALT level against the intact blank for the groups of animals that received the maximal course of treatment with the instrument and against the group of animals that were treated with Paracetamol but did not receive treatment with the instrument (Table 2).

Table 2

| Group No | ALT | AST | Bilirubin | Alkaline phosphatase |
|---|---|---|---|---|
| 1 | 11,4$\pm$3,0 | 15,7$\pm$4,8 | 12,3$\pm$1,5 | 130,4$\pm$22,6 |
| 2 | 18,1$\pm$4,3* | 18,5$\pm$3,8 | 14,0$\pm$2,3 | 120,0$\pm$25,6 |

(continued)

| Group No | ALT | AST | Bilirubin | Alkaline phosphatase |
|---|---|---|---|---|
| 3 (intact blank) | 9,9±2,4 | 18,2±4,9 | 12,2±1,4 | 114,0±27,1 |
| *The difference is statistically significant for p<0.05 (STATISTICA 6.0, Wilcoxon's test). | | | | |

Treatment with the BIK-NP instrument in the schedule of one session (10 seconds) a day gave the ALT levels closest to the values for the intact group in the absence of statistically significant differences. The effect may be evaluated as resulting from the positive therapeutic action of the instrument in the specified schedule.

Thus, an alleviation of the morphology of drug (Paracetamol)-induced hepatosis with normalization of the alanine amino transferase (ALT) serum level was discovered in the rats treated with the instrument in the schedule of one session a day lasting 10 seconds.

Example 13. Evaluation of the effect of an instrument comprising a crystal whose structure has captured the changes induced by the action of acoustic and electromagnetic fields of pancreas (hereinafter, the BIK-PZh instrument) in a normal condition on the course of Alloxan-induced diabetes in rat.

The experiment was carried out at the Research Center for Toxicology and Hygienic Regulations of Biomedicinals (the town of Serpukhov, Russia).

For inducing diabetes, pedigree male rats were administered with Alloxan monohydrate (from AppliChem) intraperitoneally in a dose of 100 mg/kg body weight in the form of an aqueous solution which was prepared immediately before administration.

On the 5th day following Alloxan administration, the glucose blood level was determined in the rats (after the morning feeding). Determination was carried out with an Accu-Chek Active glucometer in rat blood samples obtained after slitting the tips of rats' tails.

The rats selected for the experiments had glucose levels of above 10 mmol/1. A total of two groups, five animals in each, were formed:

- 1st group: the animals to be exposed to the instrument;
- 2nd group: positive blank (the blank procedure repeated the exposure process but with use of a phantom instrument).

Further, a 3rd group was formed of five intact animals which were kept in one room with the experimental rats under equal conditions but were not subjected to any manipulations but periodic blood sampling for glucose level measurements.

Treatment with the BIK-PZh instrument started immediately after the groups were formed. For treating an animal, the radiator of the instrument was applied to the abdominal wall in the area of projection of the pancreas, and the instrument was turned on for 10 seconds. The treatment course lasted 9 days in the schedule of 6 sessions a day. When the experiment was over (on the 10th day from the commencement of treatment), after 18-h starvation blood samples in an amount of 4 to 5 ml were taken from the femoral vein in all rats to be further used for the determination of alanine amino transferase (ALT), aspartate amino transferase (AST), bilirubin, alkaline phosphatase, and cholesterol levels. Euthanasia was followed by postmortem autopsy; the pancreas, liver chips, and a kidney were taken for histological investigations. Tissue samples fixed in buffered 10% formalin were subjected to routine treatment to obtain paraffin sections 5 $\mu$m thick, which were stained @@@ microscope. Statistic analysis was carried out using Microsoft Excel and STATISTICA 6.0 software.

Glucose blood levels in rats during the experiment are displayed in Table 3.

Table 3

| Group No | Ind. animal No | Glucose level (mmol/l) | | | | | |
|---|---|---|---|---|---|---|---|
| | | before treatment | 4th treatment day | 5th treatment day | | 9th treatment day | |
| | | | | After 18–h starvation | After glucose load | After 18–h starvation | After glucose load |
| 1 | 2 | 3 | 4 | 5 | 6 | 7 | 8 |
| Instrument | 1 | 21,4 | 7,9 | 5,9 | 8,2 | 4,9 | 7,8 |
| | 2 | 16,5 | 6,9 | 4,8 | 6,0 | 4,6 | 7,0 |
| | 3 | 14,3 | 6,6 | 4,3 | 6,6 | 4,3 | 6,8 |
| | 4 | 11,1 | 6,9 | 4,1 | 7,8 | 4,6 | 7,8 |
| | 5 | 19,6 | 7,4 | 4,2 | 8,1 | 4,2 | 7,4 |
| | Average | 16,6 | 7,1 | 4,7 | 7,3 | 4,5 | 7,4 |
| | Mean dev. | 4,1 | 0,5 | 0,7 | 1,0 | 0,3 | 0,5 |
| Phantom | 6 | >33,3* | >33,3 | 6,5 | 25,2 | 6,8 | >33,3 |
| | 7 | 13,4 | 8,2 | 3,6 | 16,4 | 4,0 | 18,2 |
| | 8 | 24,5 | 18,5 | 4,3 | 11,4 | 4,3 | 16,3 |
| | 9 | 21,1 | 21,7 | 4,4 | 22,9 | 5,2 | 28,6 |
| | 10 | 14,1 | 19,3 | 4,2 | 20,2 | 5,1 | 17,4 |
| | Average | 18,3 | 16,9 | 4,1 | 17,7 | 4,7 | 20,1 |
| | Mean dev. | 5,4 | 6,0 | 0,4 | 5 | 0,6 | 5,7 |
| Intact blank | 11 | 5,8 | 5,7 | 5,1 | 6,2' | 4,8 | 6,0 |
| | 12 | 5,2 | 5,8 | 4,5 | 6,2 | 4,5 | 6,6 |
| | 13 | 5,6 | 5,3 | 6,0 | 7,1 | 4,7 | 6,9 |
| | 14 | 5,7 | 5,7 | 4,6 | 5,9 | 4,8 | 7,0 |
| | 15 | 5,6 | 5,9 | 5,5 | 6,4 | 4,8 | 6,3 |
| | Average | 5,6 | 5,7 | 5,1 | 6,4 | 4,7 | 6,6 |
| | Mean dev. | 0,2 | 0,2 | 0,6 | 0,5 | 0,1 | 0,4 |

*The value exceeding the instrument's sensitivity (33.3 mmol/l) was rejected and not included into data processing.

Analysis of the exocrine part of the pancreas showed the suppression of the acinus secretion function in the Group 2 animals (which were treated with a phantom instrument), whereas the acini in the Group 1 rats did not show any changes against the intact animals.

Thus, the experimental study of the efficiency of the instrument in treating Alloxan-induced diabetes in rat showed the following:

(1) Repeated exposure to the instrument in a schedule of six sessions a day, 10 seconds per session, in 1-h intervals enhanced a reduction of the glucose blood level after glucose loading in experimental rats practically to the level determined in the intact animals, whereas in the positive blank group, no rapid reduction in glucose blood level was noticed.

(2) The functions of the exocrine (acinar) part of the pancreas were retained after exposure to the instrument, whereas in the animals from the positive blank group, a considerable reduction in pancreas functions was determined morphologically, expressed as decreased zymogen secretion.

Further, the invention will be supported by examples from clinical practice.

Example 14. Patient K, 35 years aged, complained of intermittent discomfort in the urethra and inguinal areas. Believed him to be sick for 6 months. Based on clinical anamnesis and laboratory data, diagnosed was chronic prostatitis complicated with nonspecific flora.

Staphylococcus epidermalis and hemolytic Streptococcus b, in amount of $10^3$ and $10^4$ CFU per milliliter, were isolated by the bacterial inoculation of the ejaculate. The cultures were sensitive to Amoxiclav, Cefepime, Linezolid, Oxacillin, Gentamicin, Ciprofloxacin, and Fuzidin.

The patient was proposed to take a course of treatment with an instrument comprising a crystal whose structure has captured the changes induced by the action of acoustic and electromagnetic fields of Amoxiclav antibiotic. Sessions, 10 second each, were carried out twice a day for 5 days. The instrument was applied to the projection of the prostate area. As a result of the treatment, microbiological study did not show the cultures detected hitherto. Clinical manifestations in the patient disappeared.

Example 15. Patient B complained of sore throat when swallowing, temperature of 37.6, weakness, and cough in the morning. Diagnosed was follicular angina. Repeated antibiotic treatment did not give alleviation. Microbiological studies determined Staphylococcus aureus microorganisms in an amount of 10/6 CFU per milliliter. Treatment was carried out with use of an instrument comprising a crystal whose structure captures the changes induced by the action of acoustic and electromagnetic fields of the same culture isolated from the patient in an inactive (suppressed) condition. Procedures were carried out six times a day, 10 seconds per session, onto projection of the right amygdale for 5 days. Microbiological inoculation of St. Aureus was performed before and after the treatment. This pathogen was determined before the treatment; after a session, staphylococcus was not found. By the end of the 2nd day, the patient felt alleviation, sore throat when swallowing disappeared, and temperature normalized. In seeing the pharynx, swelling and redness were observed to decrease; no pus tube was found. In further bacteriological inoculations, Staphylococcus aureus was not isolated.

**Claims**

1. A method for capturing the changes induced in the structure of an initial crystal by the action of acoustic and electromagnetic fields of a biological medium, the method comprising: using a crystal whose structure comprises a least one semiconductor junction, positioning the crystal on an insulating plate which is in direct contact with the biological medium placed in a container, and capturing the changes in the crystal structure by means of exposing the semiconductor junction thereof to a cut-off polarity electric pulse.

2. The method according to claim 1, **characterized in that** the insulating plate is made of a material having an acoustic wave absorption coefficient of no greater than 30 dB/cm and an electromagnetic wave absorption coefficient of no greater than 0.1 Neper.

3. The method according to any one of claims 1 and 2, **characterized in that** the semiconductor junction of the crystal is exposed to the action of a square electric pulse having an amplitude of 3 to 4 V and a duration of 0.1 to 1 second.

4. The method according to any one of claims 1 and 2, **characterized in that** a biological medium is preliminarily exposed to the action of an inactivating agent and **in that** the excitation of the crystal structure is carried out at the peak level of the field of the biological medium which is determined experimentally for the type of biological medium used under the action of the inactivating agent used.

**5.** The method according to claim 3, **characterized in that** a biological medium is preliminarily exposed to the action of an inactivating agent and **in that** the excitation of the crystal structure is carried out at the peak level of the field of the biological medium which is determined experimentally for the type of biological medium used under the action of the inactivating agent used.

**6.** A method for duplicating the changes that occurred in an initial crystal structure in a duplicate crystal structure, the method comprising: using a crystal whose structure comprises a least one semiconductor function, wherein the changes induced by the action of acoustic and electromagnetic fields of a biological medium are captured in the initial crystal structure by a method according to any one of claims 1, 2, 3, 4, and 5; positioning both crystals on one face of a water-filled hollow insulating cartridge; and then repeatedly exposing the semiconductor junction of the initial crystal to a straight-polarity reproduction pulse, after which exposing the semiconductor junction of the duplicate crystal to a capture cut-off polarity pulse.

**7.** The method according to claim 6, **characterized in that** the insulating cartridge is made of a material having an acoustic wave absorption coefficient of no greater than 30 dB/cm and an electromagnetic wave absorption coefficient of no greater than 0-1 Neper.

**8.** The method according to any one of claims 6 and 7, **characterized in that** the semiconductor junction of the crystal is exposed to the action of a square electric pulse having an amplitude of 3 to 4 V and a duration of 0.1 to 1 second.

**9.** A method for duplicating the changes that occurred in an initial crystal structure in a duplicate crystal structure, the method comprising: using crystals whose structure comprises a least one semiconductor junction, wherein the changes induced by the action of acoustic and electromagnetic fields of a biological medium are captured in the initial crystal structure by a method according to any of claims 1, 2, 3, 4, and 5; positioning the duplicate crystal on an insulating plate which is in direct contact with the biological medium placed in a container, wherein the semiconductor junction of the initial crystal is repeatedly exposed to the action of a straight-polarity reproduction pulse, the generated electric current oscillations modulate the light flux of a powerful semiconductor laser to expose the liquid medium placed in a container having a radial-symmetrical shape, after which the semiconductor junction of the duplicate crystal is exposed to the action of a capture cut-off polarity pulse.

**10.** The method according to claim 9, **characterized in that** the insulating plate is made of a material having an acoustic wave absorption coefficient of no greater than 30 dB/cm and an electromagnetic wave absorption coefficient of no greater than 0.1 Neper.

**11.** The method according to any one of claims 9 and 10, **characterized in that** the semiconductor junction of the crystal is exposed to the action of a square electric pulse having amplitude of 3 to 4 V and a duration of 0.1 to 1 second.

**12.** The method according to claim 11, **characterized in that** the preferred liquid medium is physiological saline.

**13.** A method for causing an affect on a biological object comprising: using, as the source of exposure, a crystal whose structure comprises at least one semiconductor junction, with the changes induced by the action of acoustic and electromagnetic fields of the biological medium being captured by the crystal structure by a method according to any of claims 1, 2, 3, 6, and 9; positioning the crystal on one face of a hollow insulating cartridge filled with an aqueous medium and having another face in direct contact with the biological object, and repeatedly exposing the semiconductor junction of the crystal to a straight-polarity electric pulse.

**14.** The method according to claim 1a, **characterized in that**, further, the electric current oscillations obtained at the crystal outlet modulate the light flux of a light-emitting diode to expose an aqueous medium.

**15.** The method according to any one of claims 13 and 14, **characterized in that** the insulating cartridge is made of a material having an acoustic wave absorption coefficient of no greater than 30 dB/cm and an electromagnetic wave absorption coefficient of no greater than 0.1 Neper.

**16.** A method for causing an affect on a biological object comprising: using, as the source of exposure, a crystal whose structure comprises at least one semiconductor junction, with the changes induced by the action of acoustic and electromagnetic fields of the biological medium being captured by the crystal structure by a method according to any of claims 1, 2, 3, 6, and 9, wherein the semiconductor junction of the crystal is repeatedly exposed to the action of a straight-polarity reproduction pulse and the generated electric current oscillations modulate the light flux of a light-emitting diode directed to the biological object.

17. A method for causing an affect on a biological object comprising: using, as the source of exposure, a crystal whose structure comprises at least one semiconductor junction, with the changes induced by the action of acoustic and electromagnetic fields of a biological medium being captured in the crystal structure by a method according to any of claims 1, 2, 3, 6, and 9, wherein the semiconductor junction of the crystals is repeatedly exposed to the action of a straight-polarity reproduction pulse and the generated electric current oscillations modulate the light flux of a semiconductor laser which is directed to the biological object.

18. A method for causing an affect on a human or animal body comprising: first recognizing the biological medium that is the initiator of a pathologic process, using a crystal with the changes induced by the acoustic and electromagnetic fields of this biological medium being captured in the crystal structure by a method according to any one of claims 4, 5, 6, and 9; and positioning the crystal on one face of a hollow insulating cartridge filled with an aqueous medium, wherein the cartridge is positioned on the skin of a man or an animal in the area of projection of the pathologic organ, and repeated exposure is carried out in two to six sessions a day, 10 seconds per session, for at least five days.

19. The method according to claim 18, **characterized in that**, further, the electric current oscillations obtained at the crystal outlet modulate the light flux of a light-emitting diode which is directed to the aqueous medium.

20. The method according to any one of claims 18 and 19, **characterized in that** the insulating cartridge is made of a material having an acoustic wave absorption coefficient of no greater than 30 dB/cm and an electromagnetic wave absorption coefficient of no greater than 0.1 Neper.

21. A method for causing an affect on a human or animal body comprising: first recognizing the biological medium that is the initiator of a pathologic process and using a crystal with the changes induced by the acoustic and electromagnetic fields of this biological medium being captured in the crystal structure by a method according to any one of claims 4, 5, 6, and 9, wherein the semiconductor junction of the crystal is repeatedly exposed to the action of a straight-polarity reproduction pulse, the semiconductor junction of the crystal is repeatedly exposed to the action of a straight-polarity reproduction pulse, the generated electric current oscillations modulate the light flux of a light-emitting diode which is directed to the skin of a man or an animal in the area of projection of a pathologic organ or a hollow organ, and exposure is carried out in two to six sessions a day, 10 seconds per session, for at least five days.

22. A method for causing an affect on a human or animal body comprising: first recognizing the biological medium that is the initiator of a pathologic process and using a crystal with the changes induced by the acoustic and electromagnetic fields of this biological medium being captured in the crystal structure by a method according to any one of claims 4, 5, 6, and 9, wherein the semiconductor junction of the crystal is repeatedly exposed to the action of a straight-polarity reproduction pulse, the generated electric current oscillations modulate the light flux of a semiconductor laser, which is directed to the skin of a man or an animal in the area of projection of a pathologic organ or to the surface of a hollow organ, and exposure is carried out in two to six sessions a day, 10 seconds per session, for at least five days.

23. A device for capturing the changes induced by the action of acoustic and electromagnetic fields of a biological medium in an initial crystal structure comprising at least one semiconductor junction, this device comprising a capture pulse generator the outlet whereof is connected in cut-off polarity to the semiconductor junction of the crystal mounted on an insulating plate which is in contact with the biological medium placed in a container.

24. The device according to claim 23, **characterized in that** the insulating plate is made of a material having an acoustic wave absorption coefficient of no greater than 30 dB/cm and an electromagnetic wave absorption coefficient of no greater than 0.1 Neper, for example, radiofrequency ceramics or fluoroplastic.

25. A device for duplicating the changes that occurred in an initial crystal structure in a duplicate crystal structure, the device comprising: crystals whose structure comprises at least one semiconductor junction, wherein the changes induced by the action of acoustic and electromagnetic fields of the biological medium are captured in the initial crystal structure by a method according to any one of claims 1, 2, 3, 4, 5, 6, and 9; a reproduction pulse generator connected in straight polarity to the semiconductor junction of the initial crystal; a capture pulse generator connected in cut-off polarity to the semiconductor junction of the duplicate crystal, wherein the crystals are positioned on one face of a hollow insulating cartridge filled with an aqueous medium.

26. The device according to claim 25, **characterized in that** the insulating cartridge is made of a material having an acoustic wave absorption coefficient of no greater than 30 dB/cm and an electromagnetic wave absorption coefficient of no greater than 0.1 Neper, for example, radioengineering ceramics or fluoroplastic.

27. A device for duplicating the changes that occurred in an initial crystal structure in a duplicate crystal structure, the device comprising: crystals whose structure comprises at least one semiconductor junction, wherein the changes induced by the action of acoustic and electromagnetic fields of the biological medium are captured in the initial crystal structure by a method according to any one of claims 1, 2, 3, 4, 5, 6, and 9; a reproduction pulse generator connected in straight polarity through a power amplifier to the semiconductor junction of the initial crystal the outlet whereof is connected to a powerful semiconductor laser; and a capture pulse generator connected in cut-off polarity to the semiconductor junction of the duplicate crystal, wherein the duplicate crystal is positioned on an insulating plate which is in direct contact with a liquid medium placed in a radial-symmetrical container and wherein the semiconductor laser is positioned so as to be capable of exposing the liquid medium.

28. The device according to claim 27, **characterized in that** the insulating plate is made of a material having an acoustic wave absorption coefficient of no greater than 30 dB/cm and an electromagnetic wave absorption coefficient of no greater than 0.1 Neper.

29. The device according to any one of claims 27 and 28, **characterized in that** the preferred liquid medium is physiological saline.

30. A device for causing an affect on a biological object, the device comprising an exposure source in the form of a crystal comprising at least one semiconductor junction in whose structure the changes induced by the action of acoustic and electromagnetic fields of a biological medium are captured by a method according to any one of claims 1, 2, 3, 4, 5, 6, and 9; a reproduction pulse generator the outlet whereof is connected in straight polarity to the semiconductor junction of the crystal which is positioned on one face of an insulating cartridge filled with an aqueous medium having another face in direct contact with the biological object.

31. The device according to claim 30, **characterized in that** it further comprises a light-emitting diode connected in series to the crystal so as it is capable of exposing the aqueous medium.

32. The device according to any one claims 30 and 31, **characterized in that** the insulating cartridge is made of a material having an acoustic wave absorption coefficient of no greater than 30 dB/cm and an electromagnetic wave absorption coefficient of no greater than 0.1 Neper, for example, radioengineering ceramics or fluoroplastic.

33. A device for causing an affect on a biological object, the device comprising an exposure source in the form of a crystal comprising at least one semiconductor junction in whose structure the changes induced by the action of acoustic and electromagnetic fields of a biological medium are captured by a method according to any one of claims 1, 2, 3, 4, 5, 6, and 9; and a reproduction pulse generator the outlet whereof is connected in straight polarity to the semiconductor junction of the crystal the outlet whereof is connected to a light-emitting diode for exposing the biological object.

34. The device according to claim 33, **characterized in that** it further comprises a flexible probe in the form of a light guide aligned with the light-emitting diode.

35. A device for causing an affect on a biological object, the device comprising: an exposure source in the form of a crystal comprising at least one semiconductor junction in whose structure the changes induced by the action of acoustic and electromagnetic fields of a biological medium are captured by a method according to any one of claims 1, 2, 3, 4, 5, 6, and 9; and a reproduction pulse generator the outlet whereof is connected in straight polarity to the semiconductor junction of the crystal the outlet whereof is connected to a semiconductor laser for exposing the biological object.

36. The device according to claim 35, **characterized in that** it further comprises a flexible probe in the form of a light guide aligned with the light-emitting diode.

Fig. 1

Fig. 2

Capture
pulse
generator                1

Power
supply unit              3

P-N junction of
the crystal              2

4

5

6

## Fig. 3

Insulating cartridge             12             13

Reproduction
pulse generator          7

P-N junction
of the initial crystal   8

P-N junction of the
duplicate crystal        10

Capture
pulse
generator                9

Power
supply unit              11

## Fig. 4

## Fig. 5

| 20 Power supply unit | 14 Reproduction pulse generator | 15 Power amplifier | 16 P-N junction of the initial crystal |

17 Powerful semiconductor laser

18 Capture pulse generator

19

21

22

23

**Fig. 5**

## Fig. 6

Biological object 30

Insulating cartridge 29

28

24 Reproduction pulse generator

25 Crystal

26 Light-emitting diode

27 Power supply unit

**Fig. 6**

Fig. 7

Fig. 8

Fig. 9

Fig. 10

Fig. 11

Fig. 12

| 49<br>Power<br>supply unit | → | 46<br>Reproduction<br>pulse<br>generator | → | 44<br>Crystal | → | 48<br>Semiconductor<br>laser |

Fig. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- RU 2250788
- RU 2055604
- RU 21993562000
- RU 2199356
- RU 2314849

### Non-patent literature cited in the description

- **N. D. Devyatkov ; M. B. Golant ; O. V. Betsky.** *Millimeter-Range Waves and Their Role in Vital Processes,* 1991, 3
- **H. Frohlich.** *J. Quant. Chem.,* 1968, vol. 2, 64
- Acoustoelectronic Interactions (AEI) entry in the Physical Encyclopedic Dictionary. 1984, 17
- **B. M. Milinkas ; A. A. Schuka.** Functional Acoustoelectronics. *Funktsional. Elektron.,* 2002, 59-62
- Physical Encyclopedic Dictionary. Sovetskaya Entsiklopediya, 1984, 568
- Physical Encyclopedic Dictionary. Sovetskaya Entsiklopediya, 1984, 567
- **M. A. Kitaev ; A.V. Moryashin ; S.V. Obolensky ; A.V. Yakimov ; A. S. Popov.** Manifestation of Natural Degradation of Schottky Field-Effect Transistors in Current-Voltage and 1/f Noise. *Proceedings of XXXV International Scientific and Methodological Workshop ''Noise and Degradation in Semiconductor Devices,'',* 2006, 39-44
- Acoustoelectronic Interactions entry in the Physical Encyclopedic Dictionary. Sovetskaya Entsiklopediya, 1984, 17
- **O. V. Betsky ; N. N. Lebedeva ; T. I. Kotrovskaya.** *Biomed. Tekhnol. Radioelektron.,* 2003, vol. Unusual, 37-43
- Physical Encyclopedic Dictionary. Sovetskaya Entsiklopediya, 1984, 25
- **A. L. Lantsov ; L. N. Zvorykin ; I. F. Osipov.** Digital Devices on Complementary MIS Integrated Circuits. *Radio i Svyaz,* 1983, 60-62